# EUROPÄISCHE PATENTSCHRIFT

(11) **EP 1 076 528 B1**
(45) Veröffentlichungstag und Bekanntmachung des Hinweises auf die Patenterteilung: **20.08.2003**
(21) Anmeldenummer: 00912240.9
(22) Anmeldetag: 29.02.2000
(51) Int. Cl.: A61B 18/02

(54) **KRYOGENES SYSTEM, INSBESONDERE FUR KRYOCHIRURGIE**
CRYOGENIC SYSTEM, ESPECIALLY FOR PERFORMING CRYOSURGICAL SURGERY
SYSTEME CRYOGENE, EN PARTICULIER POUR LA CRYOCHIRURGIE

(30) Priorität: 02.03.1999 AT 34799; 26.04.1999 AT 72999; 23.02.2000 AT 2792000
(43) Veröffentlichungstag der Anmeldung: 21.02.2001
(73) Patentinhaber: Korpan, Nikolai, 1190 Wien (AT); Zharkov, Jaroslav, Kiew, 252146 (UA)
(72) Erfinder: Korpan, Nikolai, 1190 Wien (AT); Zharkov, Jaroslav, Kiew, 252146 (UA)
(74) Vertreter: Babeluk, Michael, Dipl.-Ing. Mag.
(86) Internationale Anmeldenummer: AT0000053
(87) Internationale Veröffentlichungsnummer: WO00051509

(56) Entgegenhaltungen:
- WO-A-93/04647
- WO-A-98/52479
- GB-A- 2 026 324
- GB-A- 2 289 412
- SU-A- 1 102 096
- US-A- 4 269 390
- US-A- 4 345 598
- US-A- 5 674 218
- US-A- 5 709 203

## Beschreibung

Die Erfindung betrifft eine kryogene Vorrichtung gemäß dem Oberbegriff des Anspruches 1.

Kryogene Systeme werden vorwiegend als kryochirurgische Geräte für die Krebsbehandlung erfolgreich eingesetzt. Weitere medizinische Anwendungsbereiche bieten sich beispielsweise Allgemeinchirurgie, Urologie, Gynäkologie, HNO- und Augenkrankheiten, Plastische Chirurgie, Kieferchirurgie, Orthopädie, Veterinärmedizin, Phytopathologie usw.

Die Wärmeisolation bei bekannten Kryostaten dieser Gattung wird durch den Raum zwischen dem Gehäuse und inneren Behälter, der das kryogene Medium enthält, erzielt, wobei in diesem Raum ein Vakuum mit einem Restdruck vom ca. 10⁻⁴ mm Hg bis 10⁻⁶ mm Hg hergestellt wird. Ein permanentes Konstanthalten des oben genannten Restdruckes wird durch kryogene Pumpen, z.B. aktivierte Kohle oder Zeolith, die die Restgase aus dem Vakuumraum bei Gefrieren bis zu niedrigen Temperaturen, bis zu -196 °C, sorbieren, erreicht.

Verbindungsanordnungen zwischen den koaxialen Leitungen für Direkt- und Rückstrom des kryogenen Mediums für kryochirurgische Eingriffe im medizinischen Bereich, z.B. zum Verbinden eines Kryoapplikators mit einem Kryoinstrument, werden ebenso vorwiegend in den kryochirurgischen Geräten für die Krebsbehandlung eingesetzt. Durch bekannte Verbindungen dieser Gattung erfolgt das Abdichten eines kryogenen Mediums mit einer Zwischenlage, z.B. aus Kupfer.

Bekannte Geräte dieser Gattung haben den Nachteil, daß die Stabilisierung sowohl eines vorgegebenen Regimes mit Überdruckes eines kryogenen Systems für die kryogene Einwirkung auf das biologische Gewebe als auch die Dosierung des gefloßenen kryogenen Mediums zur verschiedenen Größe Arbeitsfläche des Kryoinstrumentes nicht genau und kontinuierlich dauernd erreicht werden können und damit die vorgegebene Temperatur für kryogene Einwirkung nicht präzise kontinuierlich dauernd behalten kann, sodaß die exakte garantierte Kryodestruktion des pathologischen Gewebes, insbesondere des bösartigen Gewebes, nicht gesichert ist und zu einem Rezidiv (Nachwachstum) des Tumors führt.

Der weitere Nachteil ist, dass die kryogenen Pumpen mit aktivierter Kohle oder Zeolith nach einiger Zeit mit Restgasen aufgefüllt werden und damit die Sorbtionseigenschaften verlieren. Um die Sorbtionseigenschaften wiederherzustellen, müssen die kryogenen Pumpen (aktivierte Kohle, Zeolith) entnommen, bei Vakuum unter Aufwärmung durch hohe Temperaturen wieder aktiviert und wiederum eingesetzt werden. Ferner ist es nachteilig, daß die Wärmeströme vom Flaschenhals zum inneren Behälter den Restdruck im Vakuumraum negativ beeinflussen.

Ebenso ist nachteilig bei den bekannten Vorrichtungen, dass eine Zwischenlage zum Abdichten des kryogenen Mediums nur ein paar Mal, jedoch nicht häufig verwendet werden kann.

Daher ist es Aufgabe der Erfindung, die bekannten kryogenen Systeme dahingehend zu verbessern, dass bei einem vorgegebenen Regime eines kryogenen Systems für kryogene Einwirkung auf das biologische Gewebe ein Überdruck exakt erzielt und beibehalten wird, und die Dosierung des gefloßenen kryogenen Mediums zur verschiedenen Größe Arbeitsfläche des Kryoinstrumentes präzise kontinuierlich konstant erreicht wird und damit die vorgegebene Temperatur für kryogene Einwirkung genau kontinuierlich konstant gesichert wird, sodaß die exakte garantierte Kryodestruktion des pathologischen Gewebes, insbesondere der bösartigen Geschwulst erreicht wird und ein Rezidiv (Nachwachstum) des Tumors vermieden wird. Dies wird mit den kennzeichnenden Merkmalen des Anspruches 1 bis 5 erreicht.

Die Aufgabe der Erfindung ist es auch daher, den bekannten Kryostaten dahingehend zu verbessern, daß der niedrige Druck von restlichen Gasen (ca. 10⁻⁴ bis 10⁻⁶ mm Hg) im Vakuumraum permanent konstant gehalten wird, sodaß die kryogenen Pumpen nicht wiederholt aktiviert werden müssen, und ferner die Wärmeströme vom Flaschenhals zum inneren Behälter zu vermindern. Dies wird mit den kennzeichnenden Merkmalen des Anspruches 6 und 7 erreicht.

Außerdem ist es die Aufgabe der Erfindung daher, die bekannten Verbindungen dahingehend zu verbessern, daß exaktes Abdichten eines kryogenen Mediums zwischen den koaxialen Leitungen für Direkt- und Rückstrom des kryogenen Mediums mit einer elektrischen Leitung für eine elektrische Verbindung bzw. Unterbrechung zwischen beiden Leitungen in den kryochirurgischen Geräten, z.B. bei häufigen Anwendungen verschiedener Kryoapplikatoren und Kryoinstrumente, erzielt wird. Dies wird ebenso mit den kennzeichnenden Merkmalen des Anspruches 8 bis 10 erreicht.

Die Unteransprüche zeigen den Kryostat mit der Verbindungsanordnung zwischen den koaxialen Leitungen für Direkt- und Rückstrom des kryogenen Mediums, die an dem kryogenen System angeschlossen sind.

Die Ansprüche 2 bis 5 zeigen ein kryogenes System mit einer Regelungsvorrichtung zur Steuerung der elektromagnetischen Ventile und des Heizelementes, wobei sich ein elektromagnetisches Ventil in einem Abstand von der Verbindungsanordnung sowie in der Leitung für den Rückstrom des verdampften kryogenen Mediums ein Heizelement befinden, sodass eine vorgegebene und gemessene vom Temperatursensor Temperatur bis einer Genauigkeit von ±1 °C infolge der Öffnung und des Schließen eines elektromagnetischen Ventils erzielt wird.

Die Erfindung gemäß Ansprüche 6 bis 7 stellt ein Kryostat, welcher zum perrnanenten Konstanthalten des niedrigen Druckes von restlichen Gasen findet und damit der notwendige restliche Druck gehalten wird.

Die Verbindungsanordnung zwischen den koaxialen Leitungen für Direkt- und Rückstrom des kryogenen Mediums gemäß Ansprüche 8 bis 10 ist entwickelt und wird in kryochirurgischen Geräten für kryochirurgische Eingriffe im medizinischen Bereich, z.B. zum Verbinden eines Kryoapplikators mit einem Kryoinstrument bei der Krebsbehandlung eingesetzt.

Die Erfindung wird anhand einer schematischen Darstellung der Vorrichtung näher erläutert und in der Zeichnung zeigen
**Fig. 1** ein kryogenes System im Längsschnitt;
**Fig. 2** ein Kryostat im Längsschnitt;
**Fig. 3** eine Verbindungsanordnung zwischen den koaxialen Leitungen für Direkt- und Rückstrom des kryogenen Mediums im Längsschnitt;

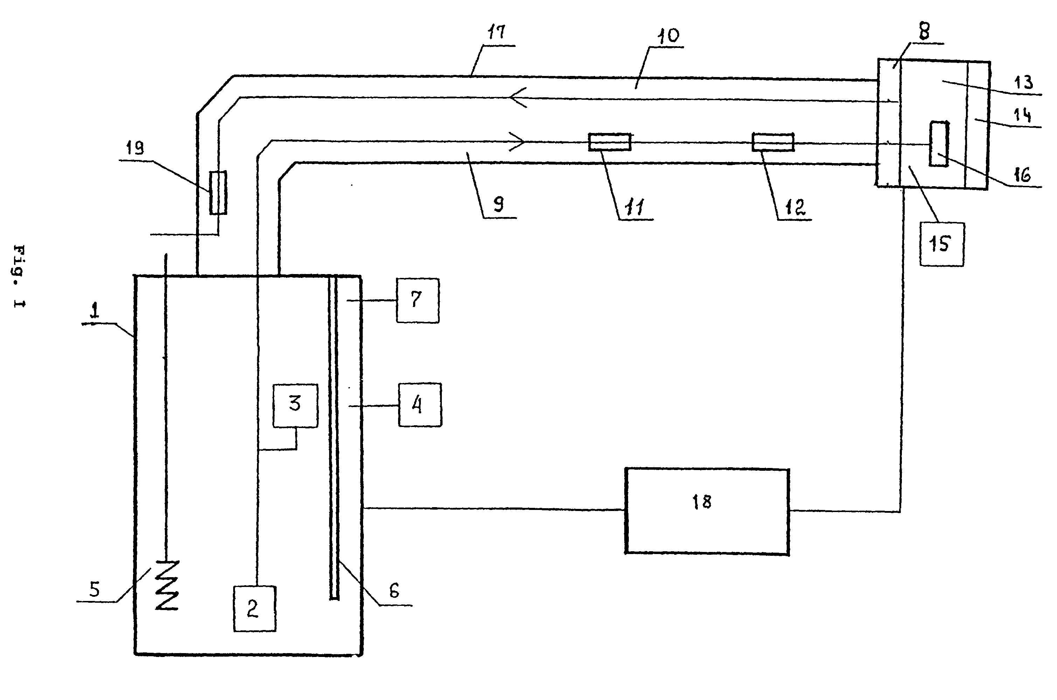

Das kryogene System für kryochirurgische Eingriffe (**Fig. 1**) im human- und vetränermedizinischen Bereich, insbesondere in der Tumorbehandlung, sowie in der Phytopathologie, besteht aus einem Kryostat (1), in dem sich ein elektromagnetisches Ventil (2) für flüssiges, kryogenes Medium, ein elektromagnetisches Ventil (3) für gasförmiges, kryogenes Medium, ein elektromagnetisches Ventil (4) zum Regeln des Überdrucks des kryogenen Mediums, eine Heizvorrichtung (5) zur Erwärmung des kryogenen Mediums, ein Sensor (6) zum Erfassen des Niveaus des kryogenen Mediums und ein Drucksensor (7) befinden, und einer Verbindungsanordnung (8) zwischen den koaxialen kryogenen Leitungen für den Direkt- (9) und Rückstrom (10) des kryogenen Mediums, wobei die Leitung (9) über das elektromagnetische Ventil (2) mit dem Kryostat (1) in Verbindung steht und in der Leitung (9) ein elektromagnetisches Ventil (11) und ein Erhitzer (12) angeordnet sind, zum Verbinden eines Kryoinstrumentes (17) mit einem Kryoansatzes (13), welcher mit einer Arbeitsfläche (14), einer Wärmeaustauschkammer (15) und einem Temperatursensor (16) versehen ist.

Zwecks der kontinuierlichen Stabilisation eines vorgegebenen Temperaturregimes eines kryogenen Systems für kryogene Einwirkung auf das biologische Gewebe und der Sicherung der garantierten Kryodestruktion, insbesondere des Krebsgewebes, befindet sich das elektromagnetische Ventil (11) in unmittelbare Nähe der Verbindungsanordnung (8). Zwischen dem Ventil (11) und der Verbindungsanordnung (8) ist weiters ein regelbares Heizelement (12) vorgesehen.

Eine Regelungsvorrichtung (18) ist zur Steuerung der elektromagnetischen Ventile (2, 3, 4 und 7) sowie des Heizelementes (12) vorgesehen, die den Überdruck des kryogenen Mediums im Kryostat (1) auf eine Genauigkeit von ± 0,1x10⁵ Pa einstellt.

Das elektromagnetische Ventil (11) befindet sich in einem Abstand von der Verbindungsanordnung (8), der kleiner ist als 1/12, vorzugsweise kleiner als ein 1/14 der Länge des Kryoinstrumentes (17).

In der Leitung (10) für den Rückstrom des verdampften kryogenen Mediums befindet sich ein Heizelement (19).

Im Kryoansatz (13) ist ein Temperatursensor (16) vorgesehen, sodass eine vorgegebene und gemessene vom Temperatursensor (16) Temperatur durch eine Stabilisierung des Druckes und kontinuierliche Dosierung des geflossenen kryogenen Mediums zur Arbeitsfläche (14) des Kryoapplikators (17) bis einer Genauigkeit von ±1 °C infolge der Öffnung und des Schließen des elektromagnetischen Ventils (11) die Arbeitsfläche (14) des Kryoansatzes (13) erzielt wird, und schließlich das kryogene Medium aus der Wärmeaustaischkammer (15) durch die kryogene Leitung (10) des Kryoinstrumentes (17) in die Atmosphäre abgeleitet wird, nachdem es im Erhitzer (19) auf Raumtemperatur aufgewärmt wurde.

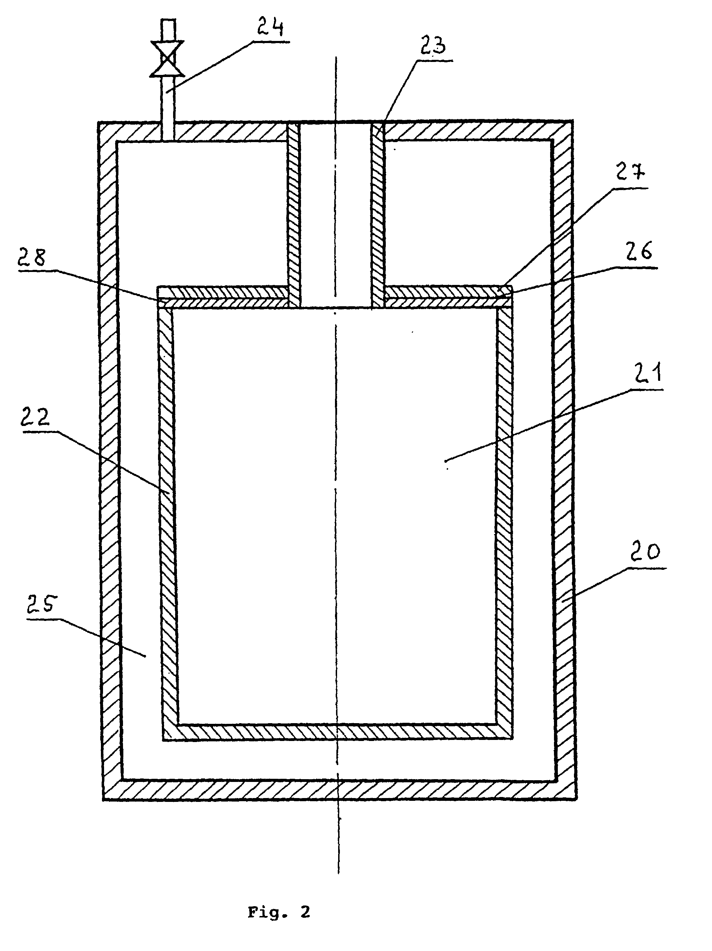

Gemäß **Fig. 2** besteht der Kryostat aus einem äußeren Gehäuse (20), inneren Behälter (21) mit einer Wand (22), die beide durch einen Flaschenhals (23) miteinander verbunden sind, ferner einer Abpumpvorrichtung (24) sowie einem Vakuumraum (25) zur Wärmeisolierung. Zum permanenten Konstanthalten des niedrigen Druckes von restlichen Gasen ist im Vakuumraum (25) die Wand (22) des inneren Behälteres (21) aus einer Aluminiumlegierung, die von der Seite des Vakuumraumes (25) durch chemische Oberflächenbehandlung, z.B. durch Ätzen, porös strukturiert, sodaß bei niedrigen Temperaturen im Vakuumraum (25) die restlichen Gase sorbiert werden und damit der notwendige restliche Druck gehalten wird, hergestellt.

Zur Verminderung von Wärmeströmen vom Flaschenhals (23) zum inneren Behälter (21) ist der Flaschenhals (23) aus einem Material mit geringer Wärmeleitung, z.B. aus rostfreiem Stahl, hergestellt ist und die obere Wandung des inneren Behälters (21) aus einer Bimetallplatte (26), deren oberer Teil (27) aus einem rostfreien Stahl und der untere Teil (28) aus einer Aluminiumlegierung besteht, wobei beide Teile (27, 28) miteinander auf der ganzen Ebene nach dem Diffusionsschweißverfahren zusammengeschweißt sind, hergestellt.

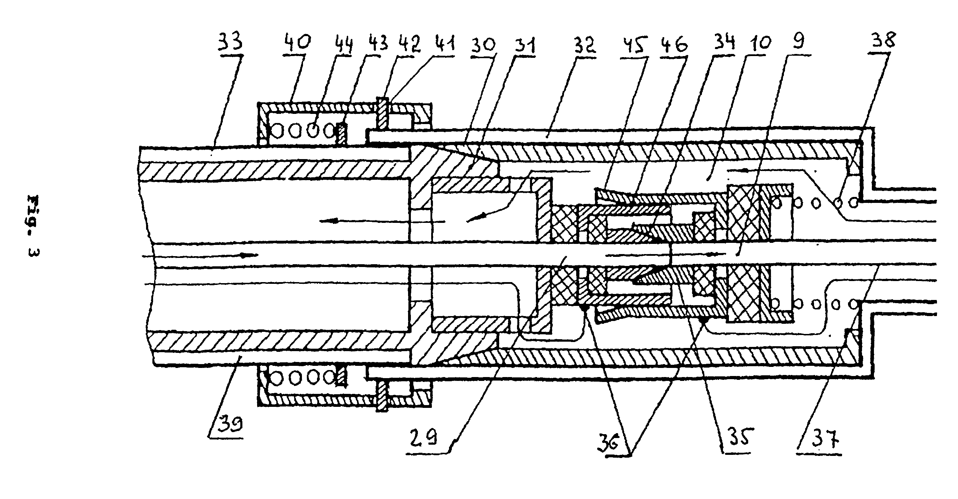

In der Verbindungsanordnung (**Fig. 3**) zwischen den koaxialen Leitungen für Direkt- (9) und Rückstrom (10) des kryogenen Mediums (29) in kryochirurgischen Geräten für kryochirurgische Eingriffe im medizinischen Bereich, z.B. zum Verbinden eines Kryoapplikators mit einem Kryoinstrument ist zur exakten Abdichtung zwischen den koaxialen Leitungen für Direkt- (9) und Rückstrom (10) des kryogenen Mediums mit Verbindungselementen (30,31,34,35) ausgestattet. Kegelstumpfförmige, miteinander korrespondierende Verbindungselemente (30,31) sind innerhalb der Leitung (10) für den Rückstrom des kryogenen Mediums (29), wobei die Leitung (10) mit äußeren vakuum- und wärmeisolierenden Hüllen (32,33) umgehen ist, und kegelstumpfförmige, miteinander korrespondierende Verbindungselemente (34,35) innerhalb der Leitung (9) für den Direktstrom des kryogenen Mediums (29), und eine elektrische Leitung (36) für eine elektrische Verbindung bzw. Unterbrechung zwischen beiden Leitungen (9,10), vorgesehen.

Die kegelstumpfförmigen Verbindungselemente (30,31) sind innerhalb der Leitung (10) für den Rückstrom des kryogenen Mediums (29) starr mit den äußeren vakuum- und wärmeisolierenden Hüllen (32,33) verbunden, und das kegelstumpfförmige Verbindungselement (34) innerhalb der Leitung für den Direktstrom (9) des kryogenen Mediums (29) unbeweglich und das starr mit einer Wand (37) der Leitung (9) verbundene kegelstumpfförmige Verbindungselement (35), jedoch in axialer Richtung gegen die Kraft einer Feder (38) verschiebbar, vorzugsweise bis 1,5 mm, angeordnet ist.

Bei geschlossener elektrischer Verbindung sind die kegelstumpfförmigen Verbindungselemente (30,31) der Leitung (10) permanent mittels einer bajonnettartigen Verbindung, die aus einem Vakuumraum (39), einem Spund (40) mit einer Fuge (41) und einer Feststellschraube (42) besteht, wobei sich zwischen dem beweglichen Spund (40) und einer fixierten Stütze (43) eine Feder (44) befindet, komprimiert.

Die kegelstumpfförmigen Verbindungselemente (30,31,34,35) sind spitzwinkelig, vorzugsweise unter einem Winkel von 12° bis 6°, ausgebildet.

Zum Verbinden der elektrischen Leitung (36) werden zwischen beiden koaxialen Leitungen (9,10) zwei hohlzylindrische Elemente (45,46), wobei das äußere zylindrische Element (45) vorzugsweise mit Anlaufflächen versehen ist, ineinander geschoben.

Das äußere zylindrische Element (45) ist zum exakten Festklemmen auf dem inneren zylindrischen Element (46) struktuiert, vorzugsweise mit 4 Riefen in einer Länge von 2/3 der gesamten Länge des Elementes (45).

Zum sicheren Festklemmen ist der äußere Durchmesser des inneren zylindrischen Elementes (46) um 0,3 mm kleiner als der innere Durchmesser des äußeren zylindrischen Elementes (45).

## Patentansprüche

1. Kryogenes System für kryochirurgische Eingriffe im human- und veterinärmedizinischen Bereich, insbesondere in der Tumorbehandlung, sowie in der Phytopathologie, zum Anschluss an ein Kryoinstrument (17) für kryochirurgische Eingriffe mit einem Kryoansatz (13), das System bestehend aus einem Kryostat (1), in dem sich ein erstes elektromagnetisches Ventil (2) für flüssiges, kryogenes Medium, ein zweites elektromagnetisches Ventil (3) für gasförmiges, kryogenes Medium, ein drittes elektromagnetisches Ventil (4) zum Regeln des Überdrucks des kryogenen Mediums, eine Heizvorrichtung (5) zur Erwärmung des kryogenen Mediums, ein Sensor (6) zum Erfassen des Niveaus des kryogenen Mediums und ein Drucksensor (7) befinden, das System ferner bestehend aus einer koaxialen Leitung für den Direkt- (9) und Rückstrom (10) des kryogenen Mediums zum und vom Kryoansatz, und einer Verbindungsanordnung (8) zwischen der koaxialen kryogenen Leitung und dem Kryoansatz (13) des Kryoinstrumentes (17), welches mit einer Arbeitsfläche (14), einer Wärmaustauschkammer (15) und einem Temperatursensor (16) versehen ist, wobei die Leitung (9) für den Direktstrom über das erste elektromagnetische Ventil (2) mit dem Kryostat (1) in Verbindung steht und in der Leitung (9) für den Direktstrom ein viertes elektromagnetisches Ventil (11) und ein Heizelement (12) angeordnet ist, **dadurch gekennzeichnet, dass** zwecks der kontinuierlichen Stabilisation eines vorgegebenen Temperaturregimes eines kryogenen Systems für kryogene Einwirkung auf das biologische Gewebe und der Sicherung der garantierten Kryodestruktion, insbesondere des Krebsgewebes, sich das vierte elektromagnetische Ventil (11) in unmittelbarer Nähe der Verbindungsanordnung (8) befindet und dass zwischen dem vierten Ventil (11) und der Verbindungsanordnung (8) ein regelbares Heizelement (12) vorgesehen ist.

2. Kryogenes System nach Anspruch 1, **dadurch gekennzeichnet, dass** eine Regelungsvorrichtung (18) zur Steuerung der elektromagnetischen Ventile (2, 3, 4 und 7) sowie des Heizelementes (12) vorgesehen ist, die den Überdruck des kryogenen Mediums im Kryostat (1) auf eine Genauigkeit von ±0,1x10⁵ Pa einstellt.

3. Kryogenes System nach einem der Ansprüche 1 oder 2, **dadurch gekennzeichnet, dass** sich das vierte elektromagnetische Ventil (11) in einem Abstand von der Verbindungsanordnung (8) befindet, der kleiner ist als 1/12, vorzugsweise kleiner als ein 1/14 der Länge des Kryoinstrumentes (17).

4. Kryogenes System nach einem der Ansprüche 1 bis 3, **dadurch gekennzeichnet, dass** sich in der Leitung (10) für den Rückstrom des verdampften kryogenen Mediums ein Heizelement (19) befindet.

5. Kryogenes System nach einem der Ansprüche 1 bis 4, **dadurch gekennzeichnet, dass** im Kryoansatz (13) ein Temperatursensor (16) vorgesehen ist, so dass eine vorgegebene und vom Temperatursensor (14) gemessene Temperatur durch eine Stabilisierung des Druckes und kontinuierliche Dosierung des geflossenen kryogenen Mediums zur Arbeitsfläche (14) des Kryoapplikators bis zu einer Genauigkeit von ±1°C infolge der Öffnung und des Schließens des vierten elektromagnetischen Ventils (11) die Arbeitsfläche (14) des Kryoansatzes (13) erzielt wird, und schließlich das kryogene Medium aus der Wärmeaustauschkammer (15) durch die kryogene Leitung (10) des Kryoinstrumentes (17) in die Atmosphäre abgeleitet wird, nachdem es im Erhitzer (19) auf Raumtemperatur aufgewärmt wurde.

6. Kryogenes System nach Anspruch 1, **dadurch gekennzeichnet, dass** es mit einem Kryostat, bestehend aus einem äußeren Gehäuse (20), inneren Behälter (21) mit einer Wand (22), die beide durch einen Flaschenhals (23) miteinander verbunden sind, ferner einer Abpumpvorrichtung (24) sowie einem Vakuumraum (25) zur Wärmeisolierung, ausgestattet ist, wobei zum permanenten Konstanthalten des niedrigen Drucks von restlichen Gasen im Vakuumraum (25) die Wand (22) des inneren Behälters (21) aus einer Aluminiumlegierung, die von der Seite des Vakuumraumes (25) durch chemische Oberflächenbehandlung, z.B. durch Ätzen, porös strukturiert ist, so dass bei niedrigen Temperaturen im Vakuumraum (25) die restlichen Gase sorbiert werden und damit der notwendige restliche Druck gehalten wird, hergestellt wird.

7. Kryogenes System nach Anspruch 6, **dadurch gekennzeichnet, dass** zur Verminderung von Wärmeströmen vom Flaschenhals (23) zum inneren Behälter (21) der Flaschenhals (23) aus einem Material mit geringer Wärmeleitung, z.B. aus rostfreiem Stahl, hergestellt ist und die obere Wandung des inneren Behälters (21) aus einer Bimetallplatte (26), deren oberer Teil (27) aus einem rostfreien Stahl und der untere Teil (28) aus einer Aluminiumlegierung besteht, wobei beide Teile (27, 28) miteinander auf der ganzen Ebene nach dem Diffusionsschweißverfahren zusammengeschweißt sind, hergestellt sind.

8. Kryogenes System nach einem der Ansprüche 1 bis 7, **dadurch gekennzeichnet, dass** eine Verbindungsanordnung zwischen den koaxialen Leitungen für den Direktstrom (9) und den Rückstrom (10) des kryogenen Mediums (29) in kryochirurgischen Geräten für kryochirurgische Eingriffe, beispielsweise zum Verbinden eines Kryoapplikators mit einem Kryoinstrument vorgesehen ist, dass zur exakten Abdichtung zwischen den koaxialen Leitungen für den Direktstrom (9) und dem Rückstrom (10) des kryogenen Mediums innerhalb dieser Leitungen (9, 10) kegelstumpfförmige, vorzugsweise unter einem Winkel zwischen 6° und 12° ausgebildete Verbindungselemente (30, 31; 34, 35) vorgesehen sind, dass die Leitung (10) mit äußeren vakuum- und wärmeisolierenden Hüllen (32, 33) umgeben ist und dass eine elektrische Leitung (36) für eine elektrische Verbindung bzw. Unterbrechung zwischen den beiden Leitungen (9, 10) vorgesehen ist.

9. Kryogenes System nach Anspruch 8, **dadurch gekennzeichnet, dass** die kegelstumpfförmigen Verbindungselemente (30, 31) innerhalb einer Leitung (10) für den Rückstrom des kryogenen Mediums (29) starr mit den äußeren vakuum- und wärmeisolierenden Hüllen (32, 33) verbunden sind, und das kegelstumpfförmige Verbindungselement (34) innerhalb der Leitung für den Direktstrom (9) des kryogenen Mediums (29) unbeweglich und das starr mit einer Wand (37) der Leitung (9) verbundene kegelstumpfförmige Verbindungselement (35), jedoch in axialer Richtung gegen die Kraft einer Feder (38) verschiebbar, vorzugsweise bis 1,5 mm, angeordnet sind und dass bei geschlossener elektrischer Verbindung die kegelstumpfförmigen Verbindungselemente (30, 31) der Leitung (10) permanent mittels einer bajonettartigen Verbindung, die aus einem Vakuumraum (39), einem Spund (40) mit einer Fuge (41) und einer Feststellschraube (42) besteht, wobei sich zwischen dem beweglichen Spund (40) und einer fixierten Stütze (43) eine Feder (44) befindet, komprimiert sind.

10. Kryogenes System nach einem der Ansprüche 8 oder 9, **dadurch gekennzeichnet, dass** die beiden koaxialen Leitungen für den Direktstrom (9) und Rückstrom (10) des kryogenen Mediums (29) zur Verbindung mit der elektrischen Leitung (36) zwei hohlzylindrische Elemente (45, 46) aufweisen, die aufeinander schiebbar sind und vorzugsweise mit Anlaufflächen versehen sind und dass das äußere hohlzylindrische Elemente (45) zum exakten Festklemmen auf dem inneren hohlzylindrischen Element (46) strukturiert ist und vorzugsweise vier Riefen in einer Länge von etwa zwei Drittel der gesamten Länge des äußeren hohlzylindrischen Elementes (45) aufweist und dass zum sicheren Festklemmen der äußere Durchmesser des inneren hohlzylindrischen Elementes (46) um etwa 0,3 mm kleiner ist als der innere Durchmesser des äußeren hohlzylindrischen Elementes (45).

## Claims

1. A cryogenic system for cryosurgical interventions in the area of human and veterinary medicine, especially in the treatment of tumours, as well as in phytopathology, for adjunction to a cryoinstrument (17) for cryosurgical interventions, with a cryoadjunct (13), said system comprising a cryostat (1) including a first electromagnetic valve (2) for a liquid cryogenic medium, a second electromagnetic valve (3) for a gaseous cryogenic medium, a third electromagnetic valve (4) for regulating the excess pressure of the cryogenic medium, a heating device (5) for heating the cryogenic medium, a sensor (6) for detecting the level of the cryogenic medium and a pressure sensor (7), the system further comprising a coaxial line for the direct flow (9) and the return flow (10) of the cryogenic medium to and from the cryoadjunct, and a connecting device (8) located between the coaxial cryogenic line and the cryoadjunct (13) of the cryoinstrument (17), which cryoinstrument is provided with a working surface (14), a chamber for heat exchange (15) and a temperature detector (16), the line (9) for the direct flow communicating with the cryostat (1) via the first electromagnetic valve (2), a fourth electromagnetic valve (11) and a heating element (12) being arranged in the line (9) for the direct flow, **characterized in that** in order to continuously stabilize a determined operating temperature of a cryogenic system for cryogenic action upon biological tissue and in order to secure the guaranteed cryodestruction of the cancerous tissue in particular, the fourth electromagnetic valve (11) is located in immediate proximity to the connecting device (8) and that a controllable heating element (12) is provided between the fourth valve (11) and the connecting device (8).

2. The cryogenic system according to claim 1, **characterized in that** a regulating device (18) is provided for controlling the electromagnetic valves (2, 3, 4 and 7) as well as the heating element (12), said regulating device regulating the excess pressure of the cryogenic medium in the cryostat (1) to an accuracy of ±0.1 x 10⁵ Pa.

3. The cryogenic system according to one of the claims 1 or 2, **characterized in that** the fourth electromagnetic valve (11) is located at a distance from the connecting device (8) that is less than 1/12, preferably less than 1/14 of the cryoinstrument's (17) length.

4. The cryogenic system according to one of the claims 1 through 3, **characterized in that** a heating element (19) is arranged in the line (10) for the return flow of the evaporated cryogenic medium.

5. The cryogenic system according to one of the claims 1 through 4, **characterized in that** a temperature detector (16) is provided in the cryoadjunct (13) so that a given temperature measured by the temperature detector (14) is reached by stabilizing the pressure and by continuously proportioning the cryogenic medium flowing to the working surface (14) of the cryoapplicator to an accuracy of ±1° C as a result of the opening and closing of the fourth electromagnetic valve (11) on the working surface (14) of the cryoadjunct (13), and that finally the cryogenic medium is heated to room temperature in the heater (19) prior to being discharged out of the chamber for heat exchange (15) through the cryogenic line (10) of the cryoinstrument (17) into the atmosphere.

6. The cryogenic system according to claim 1, **characterized in that** it is fitted with a cryostat comprising an external housing (20), an inner container (21) with a wall (22), both being joined by a bottle neck (23), furthermore of a pump-down device (24) as well as of a vacuum space (25) for thermal insulation, wherein, in order to keep permanently constant the low pressure of residual gases in the vacuum space (25), the wall (22) of the inner container (21) is made of an aluminium alloy which gets a porous structure from the side of the vacuum space (25) by way of chemical surface treatment, e.g., by etching, so that the residual gases are sorbed at low temperatures in the vacuum space (25), thus allowing the necessary residual pressure to be maintained.

7. The cryogenic system according to claim 6, **characterized in that**, to reduce the heat streams from the bottle neck (23) toward the inner container (21), the bottle neck (23) is formed of a low thermal conductivity material, e.g., of stainless steel, and the upper wall of the inner container (21) of a bimetallic plate (26), whose upper portion (27) is made of a stainless steel and the lower portion (28) of an aluminium alloy, both portions (27, 28) being diffusion bonded over the entire plane.

8. The cryogenic system according to one of the claims 1 through 7, **characterized in that** there is provided a connection device between the coaxial lines for the direct flow (9) and for the return flow (10) of the cryogenic medium (29) in cryosurgical instruments for cryosurgical interventions e.g., for connecting a cryoapplicator with a cryoinstrument, that truncated connecting elements (30, 31; 34, 35), which are preferably formed at an angle of between 6° and 12°, are provided for the accurate sealing between the coaxial lines for the direct flow (9) and for the return flow (10) of the cryogenic medium within said lines (9, 10), that the line (10) is surrounded with external, vacuum and thermally isolating cases (32, 33), and that an electric line (36) is provided for electrical connection or interruption between the two lines (9, 10).

9. The cryogenic system according to claim 8, **characterized in that** the truncated connecting elements (30, 31) are rigidly connected to the external vacuum and thermally isolating cases (32, 33) inside a line (10) for the return flow of the cryogenic medium (29) and that the truncated connecting element (34) is immovably disposed within the line for the direct flow (9) of the cryogenic medium (29) and the truncated connecting element (35), which is rigidly connected to a wall (37) of the line (9), is however slidable in axial direction against the force of a spring (38), preferably by a distance of up to 1,5 mm, and that, with a closed electrical connection, the truncated connecting elements (30, 31) of the line (10) are permanently compressed by means of a bayonet-type coupling consisting of a vacuum space (39), a tongue (40) with a joint (41) and a locking screw (42), a spring (44) being located between the movable tongue (40) and a stationary support (43).

10. The cryogenic system according to one of the claims 8 or 9, **characterized in that** the two coaxial lines for the direct flow (9) and for the return flow (10) of the cryogenic medium (29) are provided with two hollow cylindrical elements (45, 46) for connecting the electric line (36), which hollow cylindrical elements are slidable into one another and are preferably provided with stop faces, and that the external hollow cylindrical element (45) is structured for accurate clamping onto the internal hollow cylindrical element (46) and preferably has four furrows with a length amounting to about two thirds of the overall length of the external hollow cylindrical element (45) and that, for secure clamping, the outer diameter of the internal hollow cylindrical element (46) is smaller by about 0.3 mm than the inner diameter of the external hollow cylindrical element (45).

## Revendications

1. Système cryogénique pour des interventions cryochirurgicales dans le domaine de la médecine humaine et vétérinaire, en particulier dans le traitement des tumeurs, ainsi que dans la phytopathologie, pour raccordement à un cryo-instrument (17) pour effectuer des interventions cryochirugircales avec un embout cryogénique (13), le système étant composé d'un cryostat (1), dans lequel se trouvent une première soupape électromagnétique (2) pour un fluide cryogénique liquide, une deuxième soupape électromagnétique (3) pour un fluide cryogénique gazeux, une troisième soupape électromagnétique (4) pour la régulation de la surpression du fluide cryogénique, un dispositif de chauffage (5) pour le chauffage du fluide cryogénique, un capteur (6) pour la détection du niveau de fluide cryogénique et un capteur de pression (7), le système étant en outre composé d'une conduite coaxiale pour l'écoulement aller (9) et de retour (10) du fluide cryogénique vers et depuis l'embout cryogénique, et d'un dispositif de liaison (8) entre la conduite cryogénique axiale et l'embout cryogénique (13) du cryoinstrument (17), qui est muni d'une surface de travail (14), d'une chambre d'échange thermique (15) et d'un capteur de température (16), la conduite (9) pour l'écoulement aller étant reliée au cryostat (1) par la première soupape électromagnétique (2), et dans la conduite (9) étant disposée pour l'écoulement aller une quatrième soupape électromagnétique (11) et un élément chauffant (12), **caractérisé en ce que**, dans le but d'une stabilisation continue d'un régime de température prédéterminé d'un système cryogénique, prévu pour une intervention cryogénique sur un tissu biologique et, pour assurer la cryo-destruction réussie, en particulier du tissu cancéreux, la quatrième soupape électromagnétique (11) se trouve à proximité immédiate du dispositif de liaison (8), et **en ce qu'**un élément chauffant (12) réglable est prévu entre la quatrième soupape (11) et le dispositif de liaison (8).

2. Système cryogénique selon la revendication 1, **caractérisé en ce qu'**un dispositif de régulation (18) est prévu pour commander les soupapes électromagnétiques (2, 3, 4 et 7) ainsi que l'élément chauffant (12), le dispositif de régulation réglant la surpression du fluide cryogénique au cryostat (1), avec une précision de ±0,1x10⁵ Pa.

3. Système cryogénique selon l'une des revendications 1 ou 2, **caractérisé en ce que** la quatrième soupape électromagnétique (11) se trouve à une distance du dispositif de liaison (8) inférieure à 1/12, de préférence inférieure à 1/14 de la longueur du cryo-instrument (17).

4. Système cryogénique selon l'une des revendications 1 à 3, **caractérisé en ce qu'**un élément chauffant (19) se trouve dans la conduite (10) pour l'écoulement de retour du fluide cryogénique évaporé.

5. Système cryogénique l'une des revendications 1 à 4, **caractérisé en ce que**, dans l'embout cryogénique (13), est prévu un capteur de température (16), de manière qu'une température prédéterminée, mesurée par le capteur de température (16), soit atteinte, par une stabilisation de la pression et un dosage continu du fluide cryogénique véhiculé, à la surface de travail (14) de l'applicateur cryogénique (13), par une stabilisation, jusqu'à une précision de ± 11°C, par suite de l'ouverture et de la fermeture de la quatrième soupape électromagnétique (11), et **en ce que** le fluide cryogénique sortant de la chambre d'échange thermique (15) est dérivé vers l'atmosphère par la conduite cryogénique (10) du cryo-instrument (17), après avoir été chauffé à la température ambiante dans l'élément chauffant (19).

6. Système cryogénique selon la revendication 1, **caractérisé en ce qu'**il est équipé d'un cryostat, composé d'un boîtier extérieur (20), d'un récipient intérieur (21) avec une paroi (22), les deux étant reliés ensemble par un col de bouteille (23), en outre il est équipé d'un dispositif d'évacuation par pompage (24) ainsi que d'une enceinte à vide (23) pour produire l'isolation thermique, où, pour maintenir à une valeur constante en permanence la faible pression des gaz résiduels dans l'enceinte à vide (25), la paroi (22) du récipient intérieur (21) est fabriquée en alliage d'aluminium, munie d'une structure poreuse depuis le côté de l'enceinte à vide (25), ceci étant obtenu par un traitement chimique de surface, par exemple par gravure, de sorte que, aux basses températures dans l'enceinte à vide (25), les gaz résiduels soient sorbés et qu'ainsi la pression résiduelle nécessaire soit maintenue.

7. Système cryogénique selon la revendication 6, **caractérisé en ce que**, pour diminuer les flux thermiques entre le col de bouteille (23) vers le récipient intérieur (21), le col de bouteille (23) est fabriqué en un matériau ayant une faible conductivité thermique, par exemple en acier inoxydable, et la paroi supérieure du récipient intérieur (21) est formée d'une plaque bimétallique (26), dont la partie supérieure (27) est réalisée en acier inoxydable et la partie inférieure (28) en un alliage d'aluminium, les deux parties (27, 28) étant assemblées par soudage sur la totalité du plan, en utilisant un procédé de soudage par diffusion.

8. Système cryogénique selon l'une des revendications 1 à 7, **caractérisé en ce qu'**un dispositif de liaison est prévu entre les conduites coaxiales pour l'écoulement aller (9) et l'écoulement de retour (10) du fluide cryogénique (29), dans des appareils cryo-chirurgicaux pour des interventions cryo-chirurgicales, par exemple pour la liaison d'un cryo-applicateur à un cryo-instrument, **en ce que**, pour obtenir une étanchéité exacte entre les conduites coaxiales pour le courant aller (9) et le courant de retour (10) du fluide cryogénique, à l'intérieur de ces conduites (9, 10) sont prévus des éléments de liaison (30, 31; 34, 35) à forme tronconique, de préférence conformés sous un angle compris entre 6° et 12°, **en ce que** la conduite (10) est entourée de gaines extérieures (32, 33) isolant du vide et de la chaleur et **en ce qu'**une ligne électrique (36), prévue pour une liaison ou une interruption électrique, est prévue entre les deux conduites (9, 10).

9. Système cryogénique selon la revendication la revendication 8, **caractérisé en ce que** les éléments de liaison (30, 31) tronconiques, à l'intérieur d'une conduite (10) prévue pour l'écoulement de retour du fluide cryogénique (29), sont reliés rigidement aux gaines extérieures (32, 33) isolant du vide et de la chaleur, et l'élément de liaison (34) tronconique étant disposé à l'intérieur de la conduite prévue pour l'écoulement aller (9) du fluide cryogénique (19), de façon à être immobile, et l'élément de liaison (35) tronconique, relié rigidement à une paroi (37) de la conduite (9), est isolé de façon déplaçable cependant en direction axiale, à l'encontre de la force d'un ressort (38), de préférence jusqu'à une course de 1, 5 mm, et **en ce que**, lorsque la liaison électrique est fermée, les éléments de liaison (30, 31) tronconique de la conduite (10) sont comprimés en permanence à l'aide d'un raccord de type à baïonnette, formé d'un enceinte à vide (39), d'un manchon (40) avec un joint de séparation (31) et une vis de réglage (42), entre le manchon (40) mobile et un appui (43) fixe étant monté un ressort (44).

10. Système cryogénique selon l'une des revendications 8 ou 9, **caractérisé en ce que** les deux conduites coaxiales prévues pour l'écoulement aller (9) et l'écoulement de retour (10) du fluide cryogénique (29) présentent, pour assurer la liaison à la ligne électrique (36), deux éléments (45, 46) cylindriques creux, enfilables l'un sur l'autre et, de préférence, munis de surfaces de franchissement, et **en ce que** l'élément extérieur (45) cylindrique creux est structuré pour obtenir un blocage par serrage exact sur l'élément intérieur (46) cylindrique creux et, de préférence, quatre cannelures à une longueur d'environ deux tiers de la longueur totale de l'élément extérieur (45) cylindrique creux, et **en ce que**, pour assurer un blocage par serrage sûr, le diamètre extérieur de l'élément intérieur (46) cylindrique creux est inférieur, d'environ 0,3 mm, au diamètre intérieur de l'élément extérieur (45) cylindrique creux.
